# EUROPÄISCHE PATENTSCHRIFT

(11) **EP 1 077 979 B1**
(45) Veröffentlichungstag und Bekanntmachung des Hinweises auf die Patenterteilung: **06.07.2005**
(21) Anmeldenummer: 99920805.1
(22) Anmeldetag: 28.04.1999
(51) Int. Cl.: C07D 471/04, C07B 57/00

(54) **VERFAHREN ZUR HERSTELLUNG VON (S,S)-BENZYL-2,8-DIAZABICYCLO(4.3.0)NONAN**
METHOD FOR PRODUCING (S,S)-BENZYL-2,8-DIAZABICYCLO 4.3.0]NONANE
PROCEDE DE PREPARATION DE (S,S)-BENZYLE-2,8-DIAZABICYCLO 4.3.0]NONANE

(30) Priorität: 11.05.1998 DE 19821039
(43) Veröffentlichungstag der Anmeldung: 28.02.2001
(73) Patentinhaber: Bayer HealthCare AG, 51368 Leverkusen (DE)
(72) Erfinder: FEY, Peter, D-42111 Wuppertal (DE)
(86) Internationale Anmeldenummer: PCT/EP1999/002860
(87) Internationale Veröffentlichungsnummer: WO 1999/058532

(56) Entgegenhaltungen:
- EP-A- 0 409 044
- EP-A- 0 550 903
- EP-A- 0 591 808
- EP-A- 0 787 715
- WO-A-92/13858
- DE-A- 4 234 330
- GB-A- 354 975
- US-A- 5 686 614

## Beschreibung

Die vorliegende Erfindung betrifft ein Verfahren zur Herstellung von (S,S)-8-Benzyl-2,8-diazabicyclo[4.3.0]nonan [(S,S)-Benzylpyrrolopiperidin] der Formel 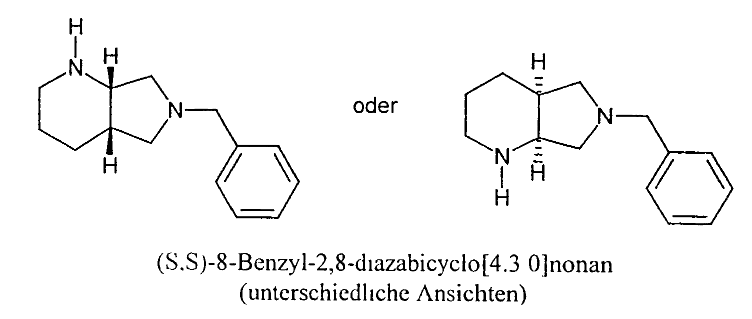 im folgenden auch als (S,S)-Benzylpyrrolopiperidin bezeichnet, durch Enantiomerentrennung eines Gemisches von (S,S)-8-Benzyl-2,8-diazabicyclo-[4.3.0]nonan und (R,R)-8-Benzyl-2,8-diazabicyclo[4.3.0]nonan. (S,S)-8-Benzyl-2,8-diazabicyclo-[4.3.0]nonan stellt ein wertvolles Zwischenprodukt für die Herstellung des (S,S)-2,8-diazabicyclo[4.3.0]nonan dar: 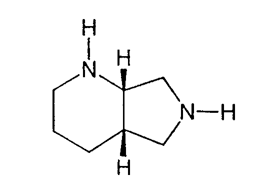 das seinerseits für die Herstellung des Antibiotikums Moxifloxacin (INN) verwendet wird:

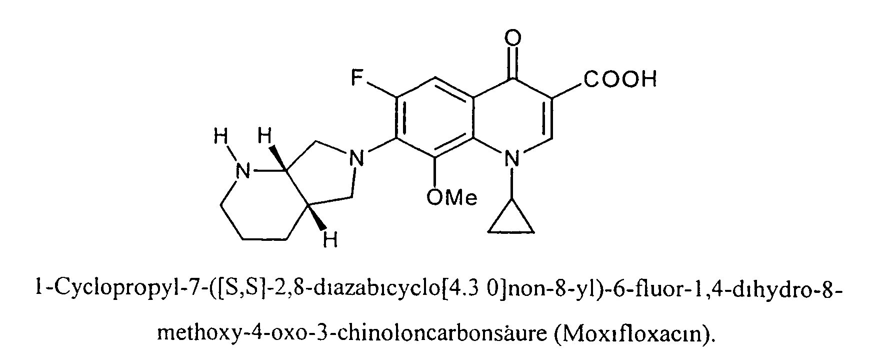

In der EP-A-0 350 733 ist die Herstellung des racemischen cis-(S,S/R,R)-8-Benzyl-2,8-diazabicyclo[4.3.0]nonans beschrieben.

Die EP-A-0 550 903 beschreibt verschiedene Verfahren zur Enantiomerentrennung des racemischen cis-(S,S/R,R)-8-Benzyl-2,8-diazabicyclo[4.3.0]nonans mit D-(-)-bzw. L-(+)-Weinsäure (Beispiel A). Diese Verfahren werden in Dimethylformamid (DMF) als Lösungsmittel durchgeführt, worin das Racemat gelöst wird So fällt z.B. gemäß Methode V nach Zugabe einer Lösung von 0,5 Äquivalenten L-(+)-Weinsäure in DMF zu einer Lösung von einem Äquivalent des cis-8-Benzyl-2,8-diazabicyclo-[4.3.0]nonan-Racemats in DMF das (R,R)-Benzylpyrrolopiperidin-L-Tartrat aus. In einem zweiten Schritt wird durch weitere Zugabe von 0,5 Äquivalenten L-(+)-Weinsäure das gewünschte (S,S)-Benzylpyrrolopiperidin-L-(+)-Tartrat ausgefällt, anschließend aus Ethanol/Wasser reinkristallisiert. Das Tartrat wird schließlich mit Basen in das freie Amin überführt. Dieses Verfahren ist jedoch im Hinblick auf die vorliegende Aufgabenstellung nachteilig, da es die vorherige Abtrennung des nicht gewünschten R,R-Enantiomers, d.h. eine zusätzliche Operation erfordert. Die Methode I Beispiels A der EP-A-0 550 903 beschreibt ein Verfahren zur Enantiomerentrennung des racemischen cis-(S,S/R,R)-8-Benzyl-2,8-diazabicyclo-[4.3.0]nonans mit D-(-)-Weinsäure in DMF. Zwar wird hier das S,S-Enantiomer zuerst ausgefällt, eine Anwendung dieses Verfahrens im industriellen Maßstab verbietet sich jedoch aufgrund des hohen Preises der unnatürlichen D-(-)-Weinsäure.

Das in den Verfahren zur Enantiomerentrennung der EP-A-0 550 903 verwendete Lösungsmittel DMF weist jedoch verschiedene Nachteile auf. Gemäß Römpp Lexikon Chemie Version 1.3, Stuttgart/New York: Georg Thieme Verlag 1997, Stichwort "Dimethylformamid", wird DMF leicht durch die Haut resorbiert, wirkt stark haut- und schleimhautreizend und Leber- und Nierenschäden sind möglich. Der MAK-Wert (Maximale Arbeitsplatzkonzentration) beträgt daher nur 10 ppm, was besondere Schutzmaßnahmen erfordert, die zu erhöhten Herstellungskosten führen.

US 5,686,614 beschreibt die Enantiomerentrennung von racemischen Aminocarbonyl-5H-dibenzo[a,d]cyclohepten-5,10-iminen mit einem Gemisch aus Ethanol und Wasser im Verhältnis von 1 : 1. Bei diesem Verfahren kommt es jedoch während der Kristallisation zu Nachfällungen des (unerwünschten) R,R-Enantiomers. Zudem ist die Ausbeute niedrig und damit das Verfahren ist unwirtschaftlich.

Daher bestand der Wunsch nach einem Verfahren zur Herstellung des (S,S)-8-Benzyl-2,8-diazabicyclo[4.3.0]nonan, daß den Einsatz problematischer Lösungsmittel vermeidet. Des weiteren sollte ein solches Verfahren möglichst hohe Ausbeuten ergeben, um Substanzverluste zu vermeiden und möglichst wenig Stufen aufweisen.

Dem Erfinder gelang es aufgrund intensiver Untersuchungen überraschend ein neues Verfahren zur Herstellung von (S,S)-Benzylpyrrolopiperidin zu entwickeln, mit dem die Enantiomerentrennung eines Gemisches von (S,S)-8-Benzyl-2,8-diazabicyclo-[4.3.0]nonan und (R,R)-8-Benzyl-2,8-diazabicyclo[4.3.0]nonan in hohen Ausbeuten in unbedenklichen Lösungsmitteln gelingt.

Gegenstand der Erfindung ist somit ein Verfahren zur Herstellung von (S,S)-8-Benzyl-2,8-diazabicyclo[4.3.0]nonan, das die Umsetzung eines Gemisches von (S,S)-8-Benzyl-2,8-diazabicyclo-[4.3.0]nonan und (R,R)-8-Benzyl-2,8-diazabicyclo-[4.3.0]nonan mit L-(+)-Weinsäure in einem Alkohol/Wasser-Gemisch umfaßt und der Alkohol ausgewählt ist aus der Gruppe, die besteht aus Ethanol, n-Propanol, Isopropanol, n-Butanol, sek.-Butanol, iso-Butanol, Isoamylalkohol und Octanol:

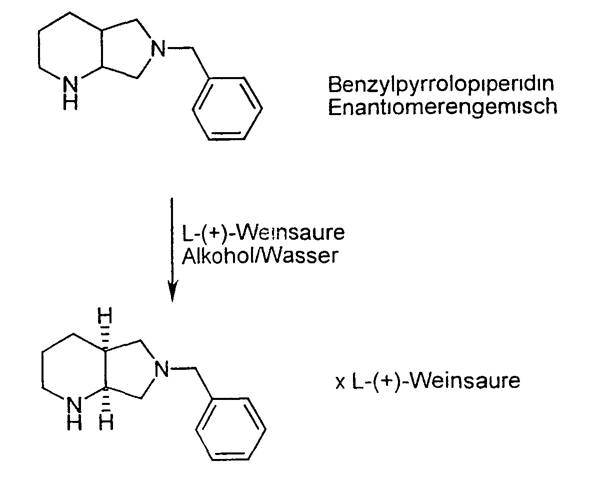

Das erfindungsgemäß eingesetzte Gemisch von (S,S)-8-Benzyl-2,8-diazabicyclo-[4.3.0]nonan und (R,R)-8-Benzyl-2,8-diazabicyclo[4.3.0]nonan umfaßt beliebige Mischungen des S,S- und R,R-Enantiomers. Bevorzugt beträgt das Verhältnis S,S-zum R,R-Isomeren von 99 : 1 bis 40 : 60, besonders bevorzugt 99 : 1 bis 50 : 50 ganz besonders bevorzugt 99 : 1 bis 60 : 40 (bezogen auf die molaren Mengen).

Das im Verfahren der Erfindung verwendete Alkohol/Wasser-Lösungsmittel-Gemisch, in dem die Bildung des (S,S)-8-Benzyl-2,8-diazabicyclo[4.3.0]nonan-L-(+)-tartrats durchgeführt wird, ist zweckmäßig ein Lösungsmittelmischung, die mindestens 40 Volumen-%, bevorzugt mindestens 50 Volumen-% einer Mischung von mindestens einem Alkohol und Wasser, bezogen auf das Gesamtvolumen des eingesetzten Lösungsmittels enthält.

In dem erfindungsgemäß verwendeten Alkohol/Wasser-Lösungsmittel-Gemisch können neben Alkohol und Wasser weitere Lösungsmittel in einer Menge von bis zu 60 Vol.-%, bevorzugt bis zu 50 Vol.-% vorhanden sein.

Das Volumen-Verhältnis Alkohol zu Wasser liegt zweckmäßig in einem Bereich von 4:1 bis 20:1, bevorzugt in einem Volumen-Verhältnis von etwa 5 (Alkohol) zu 1 (Wasser).

Die erfindungsgemäß im Lösungsmittelgemisch verwendeten Alkohole sind bevorzugt ein oder mehrere aliphatische, geradkettige oder verzweigtkettige, primäre, sekundäre oder tertiäre (C₂-C₈)-Alkohole. Diese werden beispielsweise ausgewählt aus der Gruppe, die besteht aus Ethanol, n-Propanol, Isopropanol, n-Butanol, sek.-Butanol, iso-Butanol, Isoamylalkohol und Octanol. Bevorzugt ist die Verwendung von n-, sek- oder iso-Butanol und Ethanol. Ganz besonders bevorzugt sind n-Butanol, iso-Butanol und Ethanol.

Lösungsmittel, die in dem erfindungsgemäß verwendeten Alkohol/Wasser-Lösungsmittel-Gemisch neben Alkohol und Wasser als weitere Lösungsmittel mit bis zu 60 Vol.-% (bezogen auf die Gesamtlösungsmittelmenge), bevorzugt bis zu 50 Vol.-% vorliegen können, werden beispielsweise ausgewählt aus Toluol, Xylol, Cyclohexan, Essigester, Methyl-tert.-butylether etc., wobei Toluol bevorzugt ist. Im Hinblick auf eine der oben beschriebenen Aufgabenstellungen der vorliegenden Erfindung sollten dabei wesentliche Mengen ökologisch bedenklicher Lösungsmittel, wie z.B. DMF ausgeschlossen sein und sind bevorzugt nicht vorhanden.

Wenn das Verhältnis vom S,S. zum R,R-Isomeren im erfindungsgemäß eingesetzten Gemisch von (S,S)-8-Benzyl-2,8-diazabicyclo[4.3.0]nonan und (R,R)-8-Benzyl-2,8-diazabicyclo[4.3.0]nonan von 99 : 1 bis 60 : 40 (bezogen auf die molaren Mengen) beträgt, ist die Verwendung von Ethanol als Alkohol bevorzugt. Besonders bevorzugt wird dabei ein Alkohol/Wasser-Lösungsmittelgemisch, daß nur Ethanol und Wasser, zweckmäßig in einem Volumenverhältnis von 75 : 25 bis 95 : 5, bevorzugt 80 : 20 bis 85 : 15 enthält.

Wenn das Verhältnis vom S,S-R-Isomeren im erfindungsgemäß eingesetzten Gemisch von (S,S)-8-Benzyl-2,8-diazabicyclo-[4.3.0]nonan und (R,R)-S-Benzyl-2,8-diazabicyclo[4.3.0]nonan etwa 50 : 50 (bezogen auf die molaren Mengen) beträgt, d.h., daß praktisch das racemische Gemisch verwendet wird, ist die Verwendung von Butanol als Alkohol bevorzugt. Besonders bevorzugt wird dabei ein Alkohol/Wasser-Lösungsmittelgemisch, daß aus Butanol (n-Butanol , sek.-Butanol oder iso-Butanol) und Wasser, zweckmäßig in einem Volumenverhältnis Butanol : Wasser von 4:1 bis 20:1, bevorzugt in einem Volumen-Verhältnis von etwa 5:1, wahlweise unter Zusatz von bis zu 60 Vol.-% Toluol besteht. Ganz besonders bevorzugt ist dabei ein Alkohol/Wasser-Lösungsmittelgemisch, daß nur aus Butanol (n-Butanol, sek.- Butanol oder iso-Butanol) und Wasser, zweckmäßig in einem Volumenverhältnis Butanol : Wasser von 4:1 bis 20:1, bevorzugt in einem Volumen-Verhältnis von etwa 5 zu 1 besteht. In einer weiteren bevorzugten Ausführungsform der Erfindung kann das erfindungsgemäß eingesetzte Gemisch von (S,S)-8-Benzyl-2,8-diazabicyclo-[4.3.0]nonan und (R,R)-8-Benzyl-2,8-diazabicyclo[4.3.0]nonan, das ein Verhältnis vom S,S- zum R,R-Isomeren von etwa 50 : 50 (bezogen auf die molaren Mengen) aufweist, in einem aus Ethanol, Toluol und Wasser bestehenden Lösungsmittelgemisch umgesetzt werden, das mindestens 40 Vol.-% und maximal 60 Vol.-% Toluol enthält.

Die Lösemittelvolumen liegen für die Tartratherstellung zweckmäßig im Bereich von 2 - 8 Liter des erfindungsgemäß verwendeten Alkohol/Wasser-Lösungsmittelgemischs bezogen auf 1 kg des Gemisches von (S,S)-8-Benzyl-2,8-diazabicyclo-[4.3.0]nonan und (R,R)-8-Benzyl-2,8-diazabicyclo[4.3.0]nonan, bevorzugt bei 2 bis 4 Liter, besonders bevorzugt bei etwa 3 Liter pro 1 kg des Gemisches von (S,S)-8-Benzyl-2,8-diazabicyclo[4.3.0]nonan und (R,R)-8-Benzyl-2,8-diazabicyclo[4.3.0]-nonan.

Die L-(+)-Weinsäure wird im allgemeinen im Falle der Verwendung von racemischem Benzylpyrrolopiperidin als Einsatzmaterial zweckmäßig in einer Menge von 0,4 Äquivalenten bis 1 Äquivalent, bevorzugt mit 0,7 bis 0,85 Äquivalenten bezogen auf 1 Äquivalent des racemischen Benzylpyrrolopiperidins eingesetzt.

Im Falle der Verwendung eines Gemisches von (S,S)-8-Benzyl-2,8-diazabicyclo-[4.3.0]nonan und (R,R)-8-Benzyl-2,8-diazabicyclo[4.3.0]nonan im molaren Verhältnis von 99 : 1 zu 60 : 40 und bevorzugt bei Verwendung von Ethanol als Alkohol wird L-(+)-Weinsäure zweckmäßig in einer Menge von 0,8 Äquivalenten bis 1 Äquivalent, bevorzugt mit 0,9 bis 1 Äquivalent bezogen auf 1 Äquivalent des Gemisches von (S,S)-8-Benzyl-2,8-diazabicyclo[4.3.0]nonan und (R,R)-8-Benzyl-2,8-diazabicyclo[4.3.0]nonan eingesetzt.

In einer bevorzugten Ausführungsform der Erfindung wird das Gemisch von (S,S)-8-Benzyl-2,8-diazabicyclo[4.3.0]nonan und (R,R)-8-Benzyl-2,8-diazabicyclo[4.3.0]-nonan im Alkohol gelöst und eine Lösung der L-(+)-Weinsäure in Wasser hinzugegeben. Wahlweise kann das Gemisch von (S,S)-8-Benzyl-2,8-diazabicyclo-[4.3.0]nonan und (R,R)-8-Benzyl-2,8-diazabicyclo[4 3.0]nonan auch in einer Mischung des Alkohols und des weiteren Lösungsmittels gelöst werden und dann eine Lösung der L-(+)-Weinsäure in Wasser hinzugegeben werden. Es ist auch möglich andere Dosierungsformen bei der Durchführung des erfindungsgemäßen Verfahrens zu wählen, wie z.B. die Zugabe fester L-(+)-Weinsäure zu einer Lösung des Enantiomerengemisches in dem Alkohol/Wasser-Lösungsmittelgemischs oder das Zusammengeben einer Lösung des Enantiomerengemisches in einem Alkohol/Wasser-Lösungsmittelgemisch und einer Lösung der L-(+)-Weinsäure in einem Alkohol/Wasser-Lösungsmittelgemisch.

Die Reaktionstemperaturen liegen im Fall der Tartratherstellung in Abhängigkeit von der Wahl des Alkohol/Wasser-Lösungsmittel-Gemisches, in einem Bereich von 20°C bis 110°C, bevorzugt von 45°C bis 100°C.

Die Dauer der Umsetzung liegt zweckmäßig zwischen 1 Minute und 1 Stunde bevorzugt zwischen 5 Minuten und 20 Minuten.

Bevorzugt erfolgt nach der Umsetzung des Gemisches von (S,S)-8-Benzyl-2,8-diazabicyclo[4.3.0]nonan und (R,R)-8-Benzyl-2,8-diazabicyclo[4.3.0]nonan mit der 1-(+)-Weinsäure eine Animpfung mit (S,S)-Benzylpyrrolopiperidin-L-(+)-tartrat, bevorzugt in einem Temperaturbereich von 20°C bis 110°C, besonders bevorzugt bei 40°C bis 60°C. Unter diesen Bedingungen findet bereits die Kristallisation des (S,S)-8-Benzyl-2,8-diazabicyclo[4.3.0]nonan-L-(+)-tartrates statt. Zur Vervollständigung der Kristallisation wird abhängig vom gegebenen Lösungsmittelgemisch und der Konzentration des gebildeten Tartrates zweckmäßig in einem Temperaturbereich von 0 bis 30°C, bevorzugt bei Raumtemperatur (25°C) nachgerührt.

In einer weiteren Ausführungsform kann das bei der Umsetzung des Gemisches von (S,S)-8-Benzyl-2,8-diazabicyclo[4.3.0]nonan und (R,R)-8-Benzyl-2,8-diazabicyclo-[4.3.0]nonan mit L-(+)-Weinsäure hergestellte (S,S)-8-Benzyl-2,8-diazabicyclo-[4.3.0]nonan-L-(+)-tartrat durch Umkristallisieren aus einem Alkohol/Wasser-Gemisch weiter gereinigt werden. Der Alkohol ist dabei bevorzugt mindestens ein aliphatischer Alkohol, der besonders bevorzugt aus n-, sek-, iso-Butanol und Ethanol ausgewählt wird.

Das Volumen-Verhältnis Alkohol zu Wasser im Falle der Umkristallisation liegt bevorzugt in einem Bereich von 95:5 bis 80:20.

Das Volumen-Verhältnis Alkohol zu Wasser im Fall der Umkristallisation liegt für Ethanol bevorzugt in einem Bereich von 95:5 bis 80:20 besonders bevorzugt bei etwa 85 zu 15, sowie für Butanol bevorzugt in einem Bereich von 4:1 bis 20:1, besonders bevorzugt in einem Bereich von 5:1 bis 10:1.

Die Lösemittelvolumen für die Umkristallisation liegen zweckmäßig im Bereich von etwa 2 bis 12 Liter, bevorzugt 2,5 bis 11, besonders bevorzugt 4 bis 9 Liter Alkohol/Wasser, für Ethanol bevorzugt 2,5 bis 11 Liter Ethanol/Wasser bzw. für Butanol bevorzugt 7-12 Liter Butanol/Wasser, jeweils auf 1 kg (S,S)-8-Benzyl-2,8-diazabicyclo-[4.3.0]nonan-L-(+)-tartrat. Besonders bevorzugt sind 4-6 Liter Ethanol/Wasser bzw. 8-9 Linter Butanol/Wasser auf 1 kg (S,S)-8-Benzyl-2,8-diazabicyclo-[4.3.0]nonan-L-(+)-tartrat.

Die Temperaturen liegen für den Lösungsvorgang bei der Umkristallisation in Abhängigkeit von der Wahl des Gemisches Alkohol/Wasser in einem Bereich von 70°C bis 120°C, bevorzugt bei 78°C bis 100°C.

In einer bevorzugten Ausführungsform wird bei der Umkristallisation mit (S,S)-8-Benzyl-2,8-diazabicyclo[4.3.0]nonan-L-(+)-tartrat zweckmäßig in einem Temperaturbereich von 70°C bis 120°C, bevorzugt bei 80°C bis 90°C angeimpft.

Das bei der erfindungsgemäß durchgeführten Reaktion erhaltene (S,S)-8-Benzyl-2,8-diazabicyclo[4.3.0]nonan-L-(+)-tartrat wird ggf. nach Umkristallisation in an sich bekannter Weise durch Umsetzung mit Base(n) in das freie (S,S)-8-Benzyl-2,8-diazabicyclo[4.3.0]nonan überführt (s. z.B. EP-A-0550903, Beispiel A, Methode 1). Aus diesem kann dann z.B. durch Hydrierung in an sich bekannter Weise das(S,S)-2,8-Diazabicyclo[4.3.0]nonan erhalten werden (s. z.B. EP-A-0 350 733, Beispiel K).

### Beispiel 1

### (S,S)-Benzylpyrrolopiperidin-Tartrat

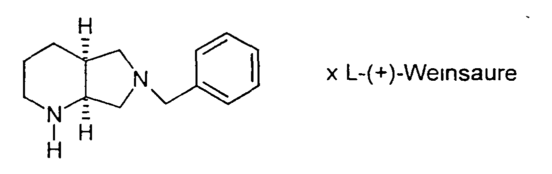

10 g (46,2 mmol) Benzylpyrrolopiperidin werden in 25 mL 1-Butanol gelöst und auf 100°C erhitzt. Es wird eine Lösung von 5,5 g (37 mmol) L-(+)-Weinsäure in 5 mL Wasser zugegeben und 5 min bei 100°C gerührt. Die Heizung wird entfernt, bei 48°C wird die Lösung mit (S,S)-Benzylpyrrolopiperidintartrat angeimpft und über Nacht bei Raumtemperatur gerührt. Die ausgefallenen Kristalle werden abgesaugt, mit wenig Butanol gewaschen und im Vakuumtrockenschrank bei 45°C zu 8,6 g (S,S)-Benzylpyrrolopiperidin-tartrat (Hydrat), 95,7 % ee (ee = enantiomeric excess; Enantiomerenüberschuß) getrocknet.

Nach Trocknung bei 80°C über Phosphorpentoxid werden 7,6 g (S,S)-Benzylpyrrolopiperidin-tartrat erhalten.
Ausbeute: 44,9 %

### Beispiel 2

### (S,S)-Benzylpyrrolopiperidin-tartrat

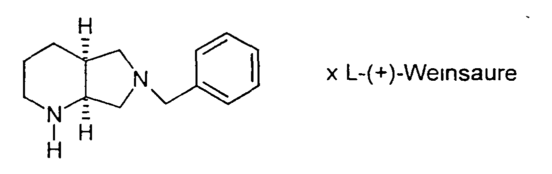

100 g (462 mmol) Benzylpyrrolopiperidin werden in 250 mL 1-Butanol gelöst. Bei Raumtemperatur wird eine Lösung von 55 g (370 mmol) L-(+)-Weinsäure in 50 mL Wasser zugegeben, wobei sich die Lösung auf ca. 46°C erwärmt. Die Lösung wird mit (S,S)-Benzylpyrrolopiperidin-tartrat angeimpft und über Nacht bei Raumtemperatur gerührt. Die ausgefallenen Kristalle werden abgesaugt, mit 50 mL Butanol/Wasser 5:1 gewaschen und im Vakuumtrockenschrank bei 45°C zu 82,9g (S,S)-Benzylpyrrolopiperidin-tartrat (Hydrat), 94,3 % ee getrocknet.

Nach Trocknung bei 80°C uber Phosphorpentoxid werden 76,2 g (S,S)-Benzylpyrrolopiperidin-tartrat erhalten.
Ausbeute: 43,8 %

### Beispiel 3

Analog Beispiel 1 wurden Versuche in folgenden Lösungsmitteln durchgeführt, wobei die L-(+)-Weinsäure in Wasser gelöst zu einer Lösung des racemischen Benzylpyrrolopiperidin in den organischen Lösungsmitteln gegeben wurde:

| **Lösemittelgemisch (Volumenverhältnis)** | **Ausbeute (% d.Th.)** | **Enantiomeren- überschuß** |
|---|---|---|
| sek.-Butanol/Wasser (5:1) | 43,7 % | 92,4 % ee SS |
| i-Butanol/Wasser (5:1) | 44,9 % | 96,0 % ee SS |
| Isoamylalkohol/Wasser (5:1) | 35,5 % | 95,4 % ee SS |
| Octanol/Wasser (5:1) | 41,1 % | 89,9 % ee SS |
| Butanol/Toluol/Wasser (5:5:1) | 41,1 % | 94,3 % ee SS |
| Toluol/Ethanol/Wasser (5:5:1) | 36,9 % | 93,2 % ee SS |

### Beispiel 4

### (S,S)-Benzylpyrrolopiperidin-Tartrat

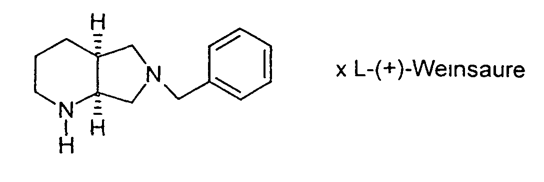

8 g (37,0 mmol) (S,S)-Benzylpyrrolopiperidin, 2 g (9,2 mmol) (R,R)-Benzylpyrrolopiperidin und 6,2 g (41,3 mmol) L-(+)-Weinsäure werden in 88 mL Ethanol/Wasser (85:15 / Vol./Vol.) gelöst und auf Rückflußtemperatur (ca. 78°C) erhitzt. Es wird 5 min bei dieser Temperatur gerührt, die Heizung wird entfernt, bei ca. 45°C wird die Lösung mit (S,S)-Benzylpyrrolopiperidin-L-(+)-tartrat angeimpft und über Nacht bei Raumtemperatur gerührt. Die ausgefallenen Kristalle werden abgesaugt, mit 15 mL Ethanol gewaschen und im Vakuumtrockenschrank bei 75°C zu 12,9 g (S,S)-Benzylpyrrolopiperidin-tartrat 98,2 % ee (ee =enantiomeric excess; Enantiomerenüberschuß) getrocknet.
Ausbeute: 94,4 % bezogen auf eingesetztes (S,S)-Benzylpyrrolopiperidin

### Beispiel 5

### (S,S)-Benzylpyrrolopiperidin-Tartrat

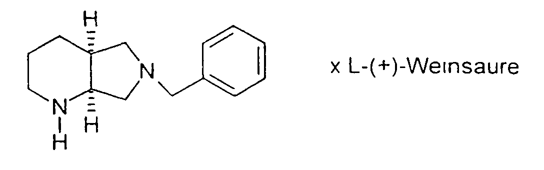

6g (27,8 mmol) (S,S)-Benzylpyrrolopiperidin, 4 g (18,4 mmol) (R,R)-Benzylpyrrolopiperidin und 5,5g (36,6 mmol) L-(+)-Weinsäure werden in 88 mL Ethanol/Wasser (85:15 / Vol./Vol.) gelöst und auf Rückflußtemperatur (ca. 78°C) erhitzt. Es wird 5 min bei dieser Temperatur gerührt, die Heizung wird entfernt, bei ca. 45°C wird die Lösung mit (S,S)-Benzylpyrrolopiperidin-L-(+)-tartrat angeimpft und über Nacht bei Raumtemperatur gerührt. Die ausgefallenen Kristalle werden abgesaugt, mit 15 mL Ethanol gewaschen und im Vakuumtrockenschrank bei 75°C zu 10,0 g (S,S)-Benzylpyrrolopiperidin-tartrat 96,9 % ee (ee = enantiomeric excess; Enantiomerenüberschuß) getrocknet.
Ausbeute: 98,1 % bezogen auf eingesetztes (S,S)-Benzylpyrrolopiperidin

### Beispiel 6

### (S,S)-Benzylpyrrolopiperidin-Tartrat (Umkristallisation)

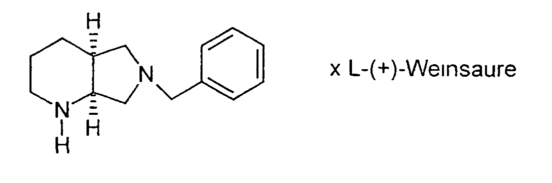

10 g des Kristallisats aus Beispiel 1 wurden in 80 mL 1-Butanol/Wasser (10:1 / Vol./Vol.) bei Siedetemperatur gelöst. Das Heizbad wurde entfernt, bei ca. 88°C wurde angeimpft und über Nacht bei Raumtemperatur gerührt. Die ausgefallenen Kristalle wurden abgesaugt, mit wenig Butanol gewaschen und bei 45°C im Vakuum getrocknet.
Ausbeute: 9,9 g, 99,6 % ee

### Beispiel 7

### (S,S)-Benzylpyrrolopiperidin-Tartrat

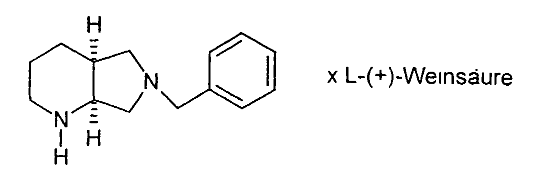

10 g des Kristallisats aus Beispiel 1 wurden in 52 mL Ethanol/Wasser (85:15 / Vol./Vol.) bei Siedetemperatur gelöst. Das Heizbad wurde entfernt, bei ca. 55°C wurde angeimpft und über Nacht bei Raumtemperatur gerührt. Die ausgefallenen Kristalle wurden abgesaugt, mit wenig Ethanol gewaschen und bei 45°C im Vakuum getrocknet.
Ausbeute: 9,9 g, 99,6 % ee

### Beispiel 8

### (S,S)-Benzylpyrrolopiperidin

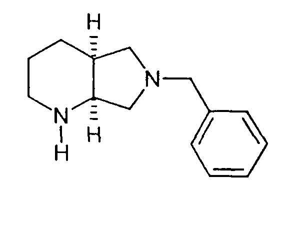

40 g des Kristallisats aus Beispiel 5 wurden in 125 ml Wasser gelöst, mit 125 ml Toluol verrührt und mit 20 ml konz. Natronlauge alkalisch gestellt. Die Toluolphase wurde abgetrennt und im Vakuum zu 23,0 g eingeengt. Das Rohprodukt wurde im Vakuum destilliert.
Ausbeute: 20,8 g
Sdp.: 104°C/0,1 mbar

## Patentansprüche

1. Verfahren zur Herstellung von (S,S)-8-Benzyl-2,8-diazabicyclo[4.3.0]nonan, **dadurch gekennzeichnet, daß** es die Umsetzung eines Gemisches von (S,S)-8-Benzyl-2,8-diazabicyclo[4.3.0]nonan und (R,R)-8-Benzyl-2,8-diazabicyclo-[4.3.0]nonan mit L-(+)-Weinsäure in einem Alkohol-Wasser-Lösungsmittelgemisch umfaßt und der Alkohol ausgewählt ist aus der Gruppe, die besteht aus Ethanol, n-Propanol, Isopropanol, n-Butanol, sek.-Butanol, iso-Butanol, Isoamylalkohol und Octanol.

2. Verfahren nach Anspruch 1, **dadurch gekennzeichnet, daß** das molare Verhältnis im eingesetzten Gemisch von (S,S)-8-Benzyl-2,8-diazabicyclo[4.3.0]-nonan zu (R,R)-8-Benzyl-2,8-diazabicyclo[4.3.0]nonan 99 : 1 bis 40 : 60 beträgt.

3. Verfahren nach Anspruch 1 oder 2, **dadurch gekennzeichnet, daß** das molare Verhältnis im eingesetzten Gemisch von (S,S)-8-Benzyl-2,8-diazabicyclo[4.3.0]-nonan zu (R,R)-8-Benzyl-2,8-diazabicyclo[4.3.0]nonan 99 : 1 bis 60 : 40 beträgt.

4. Verfahren nach einem der Ansprüche 1 bis 3, **dadurch gekennzeichnet, daß** das Alkohol-Wasser-Lösungsmittelgemisch mindestens 40 Volumen-%, bevorzugt mindestens 50 Volumen-%, einer Mischung von mindestens einem Alkohol und Wasser, bezogen auf das Gesamtvolumen des eingesetzten Lösungsmittels, enthält.

5. Verfahren nach einem der Ansprüche 1 bis 4, **dadurch gekennzeichnet, daß** das Volumen-Verhältnis Alkohol zu Wasser im Alkohol-Wasser-Lösungsmittelgemisch in einem Bereich von 4:1 bis 20:1 liegt.

6. Verfahren nach einem der Ansprüche 1 bis 5, **dadurch gekennzeichnet, daß** das Verhältnis vom S,S- zum R,R-Isomeren im eingesetzten Gemisch von (S,S)-8-Benzyl-2,8-diazabicyclo[4.3.0]nonan und (R,R)-8-Benzyl-2,8-diazabicyclo[4.3.0]-nonan von 99 : 1 bis 60 : 40 (bezogen auf die molaren Mengen) beträgt, und das Alkohol-Wasser-Lösungsmittelgemisch aus Ethanol und Wasser, bevorzugt in einem Volumenverhältnis von 75 : 25 bis 95 : 5, besteht.

7. Verfahren nach einem der Ansprüche 1, 2, 4 oder 5, **dadurch gekennzeichnet, daß** das Verhältnis vom S,S- zum R,R-Isomeren im eingesetzten Gemisch von (S,S)-8-Benzyl-2,8-diazabicyclo[4.3.0]nonan und (R,R)-8-Benzyl-2,8-diazabicyclo[4.3.0]-nonan etwa 50 : 50 (bezogen auf die molaren Mengen) beträgt, und das Alkohol-Wasser-Lösungsmittelgemisch aus Butanol (n-Butanol, sek.-Butanol oder iso-Butanol) und Wasser, zweckmäßig in einem Volumen-verhältnis Butanol : Wasser von 4:1 bis 20:1, bevorzugt in einem Volumen-Verhältnis von etwa 5 zu 1, wahlweise unter Zusatz von bis zu 60 Vol.-% Toluol, besteht.

8. Verfahren nach einem der Ansprüche 1 bis 7, **dadurch gekennzeichnet, daß** von 2 bis 8 Liter des Alkohol/Wasser-Lösungsmittelgemischs bezogen auf 1 kg des Gemisches von (S,S)-8-Benzyl-2,8-diazabicyclo[4.3.0]nonan und (R,R)-8-Benzyl-2,8-diazabicyclo[4.3.0]-nonan eingesetzt werden.

9. Verfahren nach einem der Ansprüche 1 bis 8, **dadurch gekennzeichnet, daß** ein Gemisch von (S,S)-8-Benzyl-2,8-diazabicyclo[4.3.0]nonan und (R,R)-8-Benzyl-2,8-diazabicyclo[4.3.0]-nonan in Alkohol gelöst wird und eine Lösung der L-(+)-Weinsäure in Wasser hinzugegeben wird.

10. Verfahren nach einem der Ansprüche 1 bis 9, **dadurch gekennzeichnet, daß** die Reaktionstemperatur in einem Bereich von 20°C bis 110°C, bevorzugt von 45°C bis 100°C, liegt.

11. Verfahren nach einem der Ansprüche 1 bis 10, **dadurch gekennzeichnet, daß** nach der Umsetzung des Gemisches von (S,S)-8-Benzyl-2,8-diazabicyclo[4.3.0]nonan und (R,R)-8-Benzyl-2,8-diazabicyclo[4.3.0]nonan mit der L-(+)-Weinsäure eine Animpfung mit (S,S)-8-Benzyl-2,8-diazabicyclo-[4.3.0]nonan-L-(+)-tartrat, bevorzugt in einem Temperaturbereich von 20°C bis 110°C, besonders bevorzugt bei 40°C bis 50°C, stattfindet.

12. Verfahren nach einem der Ansprüche 1 bis 11, **dadurch gekennzeichnet, daß** das bei der Umsetzung des Gemisches von (S,S)-8-Benzyl-2,8-diazabicyclo[4.3.0]nonan und (R,R)-8-Benzyl-2,8-diazabicyclo[4.3.0]nonan mit L-(+)-Weinsäure hergestellte (S,S)-8-Benzyl-2,8-diazabicyclo-[4.3.0]nonan-L-(+)-tartrat durch Umkristallisieren aus einem Alkohol/Wasser-Gemisch weiter gereinigt wird.

13. Verfahren nach Ansprüchen 1 bis 12, **dadurch gekennzeichnet, daß** das bei der Umsetzung des Gemisches von (S,S)-8-Benzyl-2,8-diazabicyclo[4.3.0]nonan und (R,R)-8-Benzyl-2,8-diazabicyclo[4.3.0]nonan mit L-(+)-Weinsäure entstandene (S,S)-8-Benzyl-2,8-diazabicyclo[4.3.0]nonan-L-(+)-tartrat, ggf. nach vorherigem Umkristallisieren, durch Umsetzung mit einer Base in das freie (S,S)-8-Benzyl-2,8-diazabicyclo[4.3.0]nonan überführt wird.

14. Verfahren zur Herstellung von (S,S)-2,8-Diazabicyclo[4.3.0]nonan, **dadurch gekennzeichnet, daß** man das nach einem der Ansprüche 1 bis 12 erhaltene (S,S)-8-Benzyl-2,8-diazabicyclo[4.3.0]nonan der hydrogenolytischen Abspaltung der Benzylgruppe unterwirft.

## Claims

1. Process for preparing (S,S)-8-benzyl-2,8-diazabicyclo[4.3.0]nonane, **characterized in that** the reaction encompasses a mixture of (S,S)-8-benzyl-2,8-diazabicyclo[4.3.0]nonane and (R,R)-8-benzyl-2,8-diazabicyclo[4.3.0]nonane with L-(+)-tartaric acid in an alcohol/water solvent mixture and the alcohol is selected from the group consisting of ethanol, n-propanol, isopropanol, n-butanol, sec-butanol, isobutanol, isoamyl alcohol and octanol.

2. Process according to Claim 1, **characterized in that** the molar ratio in the mixture used of (S,S)-8-benzyl-2,8-diazabicyclo[4.3.0]nonane to (R,R)-8-benzyl-2,8-diazabicyclo[4.3.0]nonane is 99 : 1 to 40 : 60.

3. Process according to Claim 1 or 2, **characterized in that** the molar ratio in the mixture used of (S,S)-8-benzyl-2,8-diazabicyclo[4.3.0]nonane to (R,R)-8-benzyl-2,8-diazabicyclo[4.3.0]nonane is 99 : 1 to 60 : 40.

4. Process according to any of Claims 1 to 3, **characterized in that** the alcohol/water solvent mixture contains at least 40% by volume, preferably at least 50% by volume, of a mixture of at least one alcohol and water, based on the total volume of the solvent used.

5. Process according to any of Claims 1 to 4, **characterized in that** the ratio by volume of alcohol to water in the alcohol/water solvent mixture is in a range of from 4:1 to 20:1.

6. Process according to any of Claims 1 to 5, **characterized in that** the ratio of S,S to R,R isomers in the mixture of (S,S)-8-benzyl-2,8-diazabicyclo[4.3.0]nonane and (R,R)-8-benzyl-2,8-diazabicyclo[4.3.0]nonane used is from 99 : 1 to 60 : 40 (based on the molar amounts), and the alcohol/water solvent mixture comprises ethanol and water, preferably in a ratio by volume of from 75 : 25 to 95 : 5.

7. Process according to any of Claims 1, 2, 4 or 5, **characterized in that** the ratio of S,S to R,R isomer in the mixture of (S,S)-8-benzyl-2,8-diazabicyclo[4.3.0]nonane and (R,R)-8-benzyl-2,8-diazabicyclo[4.3.0]nonane used is approximately 50 : 50 (based on the molar amounts), and the alcohol/water solvent mixture comprises butanol (n-butanol, sec-butanol or iso-butanol) and water, advantageously in a ratio by volume of butanol : water of from 4:1 to 20:1, preferably in a ratio by volume of approximately 5 to 1, optionally with addition of up to 60% by volume of toluene.

8. Process according to any of Claims 1 to 7, **characterized in that** from 2 to 8 litres of the alcohol/water solvent mixture are used, based on 1 kg of the mixture of (S,S)-8-benzyl-2,8-diazabicyclo[4.3.0]nonane and (R,R)-8-benzyl-2,8-diazabicyclo[4.3.0]nonane.

9. Process according to any of Claims 1 to 8, **characterized in that** a mixture of (S,S)-8-benzyl-2,8-diazabicyclo[4.3.0]nonane and (R,R)-8-benzyl-2,8-diazabicyclo[4.3.0]nonane is dissolved in alcohol and a solution of the L-(+)-tartaric acid in water is added.

10. Process according to any of Claims 1 to 9, **characterized in that** the reaction temperature is in a range of from 20°C to 110°C, preferably from 45°C to 100°C.

11. Process according to any of Claims 1 to 10, **characterized in that**, after the reaction of the mixture of (S,S)-8-benzyl-2,8-diazabicyclo[4.3.0]nonane and (R,R)-8-benzyl-2,8-diazabicyclo[4.3.0]nonane with the L-(+)-tartaric acid, seeding with (S,S)-8-benzyl-2,8-diazabicyclo[4.3.0]nonane L-(+)-tartrate is carried out, preferably in a temperature range of from 20°C to 110°C, particularly preferably at from 40°C to 50°C.

12. Process according to any of Claims 1 to 11, **characterized in that** the (S,S)-8-benzyl-2,8-diazabicyclo[4.3.0]nonane L-(+)-tartrate prepared in the reaction of the mixture of (S,S)-8-benzyl-2,8-diazabicyclo[4.3.0]nonane and (R,R)-8-benzyl-2,8-diazabicyclo[4.3.0]nonane with L-(+)-tartaric acid is purified further by recrystallization from an alcohol/water mixture.

13. Process according to Claims 1 to 12, **characterized in that** the (S,S)-8-benzyl-2,8-diazabicyclo[4.3.0]nonane L-(+)-tartrate formed in the reaction of the mixture of (S,S)-8-benzyl-2,8-diazabicyclo[4.3.0]nonane and (R,R)-8-benzyl-2,8-diazabicyclo[4.3.0]nonane with L-(+)-tartaric acid is, if appropriate after prior recrystallization, converted into the free (S,S)-8-benzyl-2,8-diazabicyclo[4.3.0]nonane by reaction with a base.

14. Process for preparing (S,S)-2,8-diazabicyclo[4.3.0]nonane, **characterized in that** the (S,S)-8-benzyl-2,8-diazabicyclo[4.3.0]nonane obtained according to any of Claims 1 to 12 is subjected to hydrogenolytic cleavage of the benzyl group.

## Revendications

1. Procédé de production de (S,S)-8-benzyl-2,8-diazabicyclo[4.3.0]nonane, **caractérisé en ce qu'**il comprend la réaction d'un mélange de (S,S)-8-benzyl-2,8-diazabicyclo-[4.3.0]nonane et de (R,R)-8-benzyl-2,8-diazabicyclo[4.3.0]nonane avec l'acide L-(+)-tartrique dans un solvant formé d'un mélange d'alcool et d'eau, et l'alcool est choisi dans le groupe qui est constitué de l'éthanol, du n-propanol, de l'isopropanol, du n-butanol, du sec.-butanol, de l'isobutanol, de l'alcool isoamylique et de l'octanol.

2. Procédé suivant la revendication 1, **caractérisé en ce que** le rapport molaire du (S,S)-8-benzyl-2,8-diazabicyclo[4.3.0]nonane au (R,R)-8-benzyl-2,8-diazabicyclo-[4.3.0]nonane dans le mélange utilisé va de 99:1 à 40:60.

3. Procédé suivant la revendication 1 ou 2, **caractérisé en ce que** le rapport molaire du (S,S)-8-benzyl-2,8-diazabicyclo[4.3.0]nonane au (R,R)-8-benzyl-2,8-diazabicyclo-[4.3.0]nonane dans le mélange utilisé va de 99:1 à 60:40.

4. Procédé suivant l'une des revendications 1 à 3, **caractérisé en ce que** le solvant formé d'un mélange d'alcool et d'eau contient au moins 40 % en volume, avantageusement au moins 50 % en volume, d'un mélange d'au moins un alcool et d'eau, par rapport au volume total du solvant utilisé.

5. Procédé suivant l'une des revendications 1 à 4, **caractérisé en ce que** le rapport en volume de l'alcool à l'eau dans le solvant formé d'un mélange d'alcool et d'eau se situe dans la plage de 4:1 à 20:1.

6. Procédé suivant l'une des revendications 1 à 5, **caractérisé en ce que** le rapport de l'isomère S,S à l'isomère R,R dans le mélange utilisé de (S,S)-8-benzyl-2,8-diazabicyclo[4.3.0]nonane et de (R,R)-8-benzyl-2,8-diazabicyclo-[4.3.0]nonane va de 99:1 à 60:40 (sur la base des quantités molaires) et le solvant formé d'un mélange d'alcool et d'eau est constitué d'éthanol et d'eau, avantageusement dans un rapport en volume de 75:25 à 95:5.

7. Procédé suivant l'une des revendications 1, 2, 4 ou 5, **caractérisé en ce que** le rapport de l'isomère S,S à l'isomère R,R dans le mélange utilisé de (S,S)-8-benzyl-2,8-diazabicyclo[4.3.0]nonane et de (R,R)-8-benzyl-2,8-diazabicyclo[4.3.0]nonane est d'environ 50:50 (sur la base des quantités molaires), et le solvant formé d'un mélange d'alcool et d'eau est constitué de butanol (n-butanol, sec.-butanol ou isobutanol) et d'eau, convenablement dans un rapport en volume butanol:eau de 4:1 à 20:1, avantageusement dans un rapport en volume d'environ 5 à 1, éventuellement avec addition de toluène jusqu'à 60 % en volume.

8. Procédé suivant l'une des revendications 1 à 7, **caractérisé en ce qu'**on utilise 2 à 8 litres du solvant formé d'un mélange alcool/eau sur la base de 1 kg du mélange de (S,S)-8-benzyl-2,8-diazabicyclo[4.3.0]nonane et de (R,R)-8-benzyl-2,8-diazabicyclo[4.3.0]nonane.

9. Procédé suivant l'une des revendications 1 à 8, **caractérisé en ce qu'**on dissout un mélange de (S,S)-8-benzyl-2,8-diazabicyclo[4.3.0]nonane et de (R,R)-8-benzyl-2,8-diazabicyclo[4.3.0]nonane dans l'alcool et on ajoute une solution aqueuse d'acide L-(+)-tartrique.

10. Procédé suivant l'une des revendications 1 à 9, **caractérisé en ce que** la température de réaction se situe dans un intervalle de 20°C à 110°C, avantageusement de 45°C à 100°C.

11. Procédé suivant l'une des revendications 1 à 10, **caractérisé en ce qu'**un amorçage avec du L-(+)-tartrate de (S,S)-8-benzyl-2,8-diazabicyclo[4.3.0]nonane a lieu avantageusement dans une plage de températures de 20°C à 110°C, notamment dans une plage de 40 à 50°C après la réaction du mélange de (S,S)-8-benzyl-2,8-diazabicyclo-[4.3.0]nonane et de (R,R)-8-benzyl-2,8-diazabicyclo-[4.3.0]nonane avec l'acide L-(+)-tartrique.

12. Procédé suivant l'une des revendications 1 à 11, **caractérisé en ce que** le L-(+)-tartrate de (S,S)-8-benzyl-2,8-diazabicyclo[4.3.0]nonane produit dans la réaction du mélange de (S,S)-8-benzyl-2,8-diazabicyclo[4.3.0]nonane et de (R,R)-8-benzyl-2,8-diazabicyclo[4.3.0]nonane avec l'acide L-(+)-tartrique est encore purifié par recristallisation dans un mélange alcool/eau.

13. Procédé suivant les revendications 1 à 12, **caractérisé en ce que** le L-(+)-tartrate de (S,S)-8-benzyl-2,8-diazabicyclo[4.3.0]nonane formé dans la réaction du mélange de (S,S)-8-benzyl-2,8-diazabicyclo[4.3.0]nonane et de (R,R)-8-benzyl-2,8-diazabicyclo[4.3.0]nonane avec l'acide L-(+)-tartrique est transformé en (S,S)-8-benzyl-2,8-diazabicyclo[4.3.0]nonane libre par réaction avec une base, éventuellement après recristallisation préalable.

14. Procédé de production de (S,S)-8-benzyl-2,8-diazabicyclo[4.3.0]nonane, **caractérisé en ce qu'**on soumet le (S,S)-8-benzyl-2,8-diazabicyclo[4.3.0]nonane obtenu selon l'une des revendications 1 à 12 à l'élimination par hydrogénolyse du groupe benzyle.
